# EUROPÄISCHE PATENTSCHRIFT

(11) **EP 2 480 180 B1**
(45) Veröffentlichungstag und Bekanntmachung des Hinweises auf die Patenterteilung: **09.07.2014**
(21) Anmeldenummer: 10760922.4
(22) Anmeldetag: 21.09.2010
(51) Int. Cl.: A61F 5/01

(54) **AUS ELASTISCHEM MATERIAL BESTEHENDE KNIEGELENKBANDAGE MIT ANZIEHHILFE**
KNEE ORTHOSIS MADE OF ELASTIC MATERIAL AND PROVIDED WITH A DONNING AID
GENOUILLÈRE EN MATÉRIAU ÉLASTIQUE AVEC AUXILIAIRE D'ENFILAGE

(30) Priorität: 25.09.2009 DE 202009012967 U
(43) Veröffentlichungstag der Anmeldung: 01.08.2012
(73) Patentinhaber: Bauerfeind AG, 07937 Zeulenroda-Triebes (DE)
(72) Erfinder: REINHARDT, Holger, 47906 Kempen (DE); BERENDT, Uwe, 44536 Lünen (DE)
(74) Vertreter: Puschmann Borchert Bardehle Patentanwälte Partnerschaft mbB
(86) Internationale Anmeldenummer: PCT/EP2010/005773
(87) Internationale Veröffentlichungsnummer: WO 2011/035885

(56) Entgegenhaltungen:
- WO-A1-00/49982
- DE-A1- 3 637 879

## Beschreibung

Die Erfindung bezieht sich auf eine aus elastischem Material bestehende Kniegelenkbandage mit einem der Kniescheibe zugeordneten Polster, die auf mindestens einer Seite des Polsters mit einem biegsamen, sich über die Länge der Bandage erstreckenden Stabilisierungsstab versehen ist.

Eine derartige Bandage ist in der DE 36 37 879 A1 dargestellt und beschrieben. Diese Bandage ist mit seitlich neben dem Polster angeordneten Federbandstäben ausgestattet, die sich praktisch über die gesamte Länge der Bandage erstrecken und das Kniegelenk bei dessen Beugung besonders abstützen.

Derartige Kniegelenkbandagen umspannen das Kniegelenk je nach Elastizität des Bandagenmaterials mehr oder minder stramm, was dazu führt, dass das Anziehen der Bandage und das Überstreifen über die Ferse und das Kniegelenk erschwert ist und auf jeden Fall einen erheblichen Kraftaufwand voraussetzt. Zur Erleichterung des Anziehens einer solchen Kniegelenkbandage hat man gemäß WO 00/49982 bereits vorgeschlagen, am oberen Rad der Kniegelenkbandage ein oder mehrere Schlaufen anzubringen, um in diese Schlaufen mit einem Finger einzugreifen und dann an ihnen zu ziehen, wodurch die an den Schlaufen angenähte Bandage mitgezogen und über das Knie hinweggeschleift werden kann. Hierzu ist eine sehr solide Verbindung zwischen Schlaufe und dem Material der Kniegelenksbandage erforderlich, damit die Schlaufe beim Anziehen der Bandage nicht abreißen kann.

Der Erfindung liegt die Aufgabe zugrunde, das Anlegen einer derartigen Kniegelenkbandage mit erforderlicher Sicherheit für das Bandagenmaterial zu erleichtem. Erfindungsgemäß geschieht dies dadurch, dass die Öse an ihrer dem Stabilisierungsstab abgewandten Seite eine Verdickung aufweist. Der Stabilisierungsstab wird hierbei mit doppelter Wirkung ausgenutzt, nämlich einerseits zur Stabilisierung des Kniegelenks und andererseits als Anziehhilfe, wozu der Stabilisierungsstab mit einem Griffstück versehen ist, das leicht erfassbar ist und einen auf ihn ausgeübten Zug direkt auf das Bandagenmaterial überträgt. Der Einschluss des Stabilisierungsstabes in die an die Bandage angeordnete Tasche gibt dem Stabilisierungsstab eine ausreichende feste Verbindung zu dem Material der Bandage, so dass der auf das Griffstück ausgeübte Zug sich gut über das Bandagenmaterial verteilen lässt und diese daher beim Anziehen keiner besonders hohen zusätzlichen Beanspruchung ausgesetzt ist.

Der Stabilisierungsstab wird hierbei mit doppelter Wirkung ausgenutzt, nämlich einerseits zur Stabilisierung des Kniegelenks und andererseits als Anziehhilfe, wozu der Stabilisierungsstab mit einem Griffstück versehen ist, das leicht erfassbar ist und einen auf ihn ausgeübten Zug direkt auf das Bandagenmaterial überträgt.

Beim Zug an dem Griffstück wird dieser auf diese Weise über die gesamte Länge der Bandage in diese eingeleitet.

Das Griffstück wird zweckmäßig als Öse ausgebildet, bei dem der Durchgang durch sein Loch etwa rechtwinkelig zum Bandagenmaterial liegt. Bei einer solchen Ausbildung des Griffstücks kann dieses mit einem Finger direkt erfasst werden, der dabei die Öse durchsetzt und den Zug auf diese weise bequem auf die Bandage überträgt. Das Erfassen der Öse lässt sich weiterhin dadurch erleichtern, dass diese an ihrer dem Stabilisierungsstab abgewandten Seite eine Verdickung aufweist.

In der Figur ist ein Ausführungsbeispiel der Erfindung dargestellt. Es zeigen:
- Fig. 1: eine Draufsicht auf das Knie mit angelegter Kniegelenkbandage,
- Fig. 2: das Griffstück allein mit Draufsicht auf das Loch in der Öse,
- Fig. 3: das Griffstück gemäß Fig. 2 in Seitenansicht.

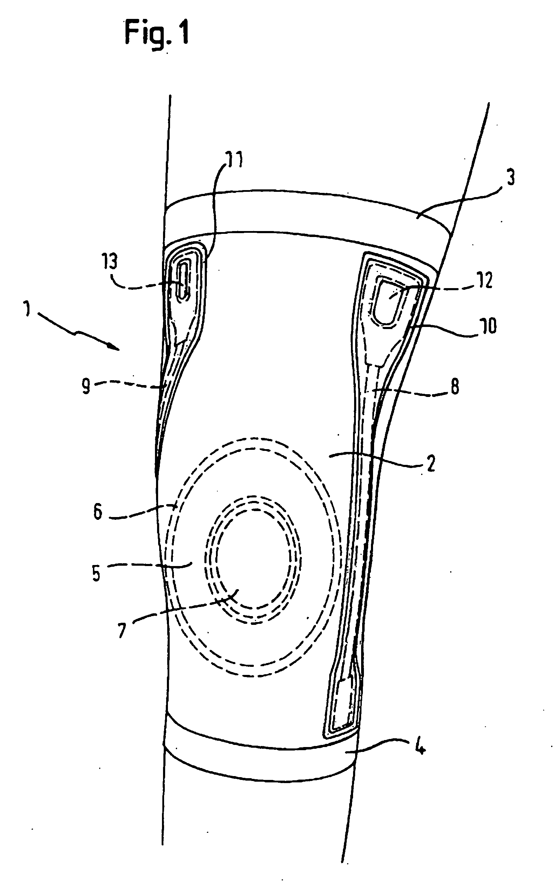

Die in der Figur 1 dargestellte Kniegelenkbandage 1 besteht aus dem aus elastischem Textilmaterial bestehenden Strumpf 2, sie ist mit den beiden Rändern 3 und 4 an ihren beiden Enden versehen, die zur Rutschsicherung der Bandage 1 beitragen. Außerdem sind diese Ränder 3 und 4 aus einem Material hergestellt, das eine geringere Spannung als der Strumpf 2 aufweist, um das Bein des Trägers an den betreffenden Stellen nur wenig einzuschnüren. Auf der Vorderseite des Kniegelenks ist in dem Strumpf 2 eine Profileinlage mit dem Polster 5 eingearbeitet, die zum Beispiel aus Schaumstoff oder Silikon bestehen kann und eine erhebliche Elastizität besitzt. Das Polster 5 ist auf der Innenseite des Strumpfes 2 durch einen Überzug abgedeckt, der an seinen Rändern 6 mit dem Strumpf 2 verbunden ist, beispielsweise durch Kleben. Das Polster 5 lässt in ihrem mittleren Teil einen Bereich frei, in dem etwa die Kniescheibe 7 hineinpasst. Die Kniescheibe 7 wird somit von dem Polster 5 umfasst. In soweit handelt es sich um eine in bekannter Weise gestaltete Kniegelenkbandage.

Neben dem Polster 5 ist die Bandage 1 mit zwei Stabilisierungsstäben 8 und 9 versehen, die sich im wesentlichen über die gesamte Länge der Bandage 1 erstrecken und die dafür sorgen, dass sich die am Bein angelegte Bandage 1 nicht hinsichtlich ihrer Längsrichtung zusammenziehen kann. Jeder der beiden Stabilisierungsstäbe 8 und 9 ist in eine auf die Bandage 1 mittels der Randzone 10 bzw. 11 an das Material der Bandage 1 angeklebte Tasche aufgenommen. Je nach gewünschter Stabilisierungsintensität kann die Bandage 1 auch nur mit einem Stabilisierungsstab versehen sein.

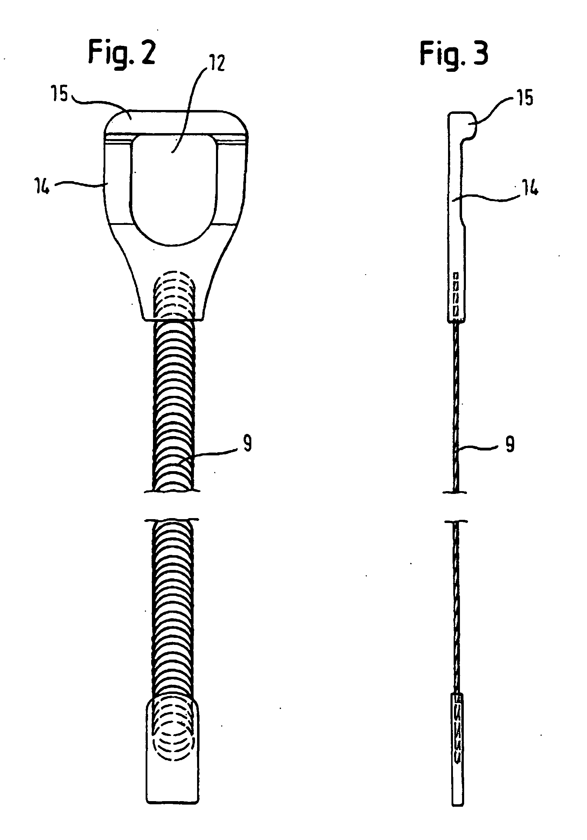

Auf die besondere Gestaltung der Stabilisierungsstäbe 8, 9 im Bereich ihres Griffstücks wird in den Figuren 2 und 3 näher eingegangen.

Jeder der beiden Stabilisierungsstäbe 8 und 9 weist an seinem oberen Ende ein eine Öse 12 bzw. 13 enthaltendes Griffstück 14 auf, welches das Erfassen der Bandage 1 bei ihrem Anziehen und Hochziehen entlang des Beines mit dem Finger ermöglicht und damit das Anziehen der Bandage 1 erleichtert, da die Bandage 1 bei einem entsprechenden Zug auf die Griffstücke 14 von diesen und dem Stabilisierungsstab 8, 9 insgesamt mitgenommen wird, wodurch es ohne weiteres möglich ist, die Bandage 1 glatt über den Fuß, die Wade und das Knie in ihre endgültige Lage zu ziehen.

Die in den Taschen enthaltenen Stabilisierungsstäbe 8 und 9 werden dadurch satt von den betreffenden Taschen umfasst, dass deren Randzone 10 und 11 jeweils als schmaler umlaufener Streifen ausgebildet ist, der direkt mit dem Material der Bandage 1 verbunden ist, zum Beispiel durch verschweißen oder durch verkleben.

Figur 2 zeigt einen Stabilisierungsstab 9 allein mit dem Griffstück 14, dass das Loch 12 umfasst. Das Griffstück 14 besteht aus einem elastischen Kunststoff, in sein unteres Ende ist der eigentliche Stabilisierungsstab 9 eingesetzt und wird dort von dem Material des Griffstücks 14 satt und Zugsicher erfasst. Der Stabilisierungsstab 9 besteht aus den flachgepressten Windungen einer Schraubenfeder, so dass sich bei axialer Strecksicherheit eine erhebliche Elastizität für ein Biegen ergibt, womit der Stabilisierungsstab 9, angebracht an der Bandage 1, sich auch biegen kann.

Figur 3 zeigt das Griffstück 14 mit eingesetztem Stabilisierungsstab 9 in Seitensicht. Diese zeigt am oberen Ende des Griffstücks 14 die Verdickung 15, die dazu dient, einem in die Öffnung 12 des Griffstücks 14 eingedrückten Finger einen guten Halt beim Anziehen der Bandage 1 zu geben.

## Patentansprüche

1. Aus elastischem Material bestehende Kniegelenkbandage (1) mit einem der Kniescheibe zugeordneten Polster, die auf mindestens einer Seite des Polsters mit einem biegsamen, sich über die Länge der Bandage erstreckenden Stabilisierungsstab (8, 9) versehen ist, **dadurch gekennzeichnet, dass** der Stabilisierungsstab (8, 9) mit einem Griffstück (14) versehen und in eine an der Bandage (1) angeordneten Tasche eingebettet ist, die über Randzonen (10, 11) und an ihrem oberhalb der Kniescheibe angeordneten Ende mit dem Material der Bandage (1) fest verbunden ist.

2. Kniegelenkbandage nach Anspruch 1, **dadurch gekennzeichnet, dass** der Stabilisierungsstab (8, 9) im wesentlichen durchgehend mit dem Material der Bandage (1) verschweißt ist.

3. Kniegelenkbandage nach Anspruch 1 oder 2, **dadurch gekennzeichnet, dass** das Griffstück (14) als Öse (12, 13) ausgebildet ist, bei dem der Durchgang durch sein Loch etwa rechtwinklig zum Bandagenmaterial liegt.

4. Kniegelenkbandage nach einem der Ansprüche 1 bis 3, **dadurch gekennzeichnet, dass** die Öse (12, 13) an ihrer dem Stabilisierungsstab (8, 9) abgewandten Seite eine Verdickung (15) aufweist.

## Claims

1. A knee orthosis (1) made of elastic material and including a pad for the kneecap, said knee orthosis having, on at least one side of said pad, an elastic stabilizing rod (8, 9) which extends along the entire length of said knee orthosis **characterized in that** said stabilizing rod (8, 9) is provided with a grab element (14) and is embedded in a pocket disposed on said orthosis (1), which pocket is firmly attached to the material of said orthosis (1) along its edge zones (10, 11) and at its end located above the kneecap.

2. The knee orthosis of claim 1 **characterized in that** said stabilizing rod (8, 9) is essentially continuously welded onto the material of said orthosis (1).

3. The knee orthosis of claims 1 or 2 **characterized in that** said grab element (14) is in the form of an eyelet (12, 13), with the passage through its the center hole extending approximately perpendicular to the material of said orthosis.

4. The knee orthosis of claims 1 to 3 **characterized in that** said eyelet (12, 13) has a thickened portion (15) on its side facing away from said stabilizing rod (8, 9).

## Revendications

1. Genouillère (1) en matériau élastique avec un rembourrage assigné à la rotule pourvu, au moins d'un côté du rembourrage, d'une tige stabilisatrice (8, 9) flexible qui s'étend sur la longueur de la genouillère, **caractérisée en ce que** la tige stabilisatrice (8, 9) est pourvue d'une poignée (14) et encastrée dans une poche, disposée sur la genouillère (1), qui est fermement rattachée au matériau de la genouillère (1) par les rebords (10, 11) et par son extrémité située au-dessus de la rotule.

2. Genouillère selon la revendication 1, **caractérisée en ce que** la tige stabilisatrice (8, 9) est essentiellement soudée de façon continue avec le matériau de la genouillère (1).

3. Genouillère selon les revendications 1 ou 2, **caractérisée en ce que** la poignée (14) est formée comme un oeillet (12, 13) dont le passage sous le trou est presque en angle droit par rapport au matériau de la genouillère.

4. Genouillère selon l'une des revendications 1 à 3, **caractérisée en ce que** l'oeillet (12, 13) présente un épaississement (15) du côté opposé à la tige stabilisatrice (8, 9).
